# EUROPEAN PATENT APPLICATION

(11) **EP 3 808 337 A1**
(43) Date of publication of application: **21.04.2021**
(21) Application number: 19819957.2
(22) Date of filing: 06.03.2019
(51) Int. Cl.: A61K 9/00, A61K 47/42, A61K 35/30, A61P 25/00

(54) **STEM CELL PREPARATION SPONGE PATCH COMPLEX FOR TREATING BRAIN DISEASE, PREPARATION METHOD THEREFOR AND APPLICATION THEREOF**

(30) Priority: 15.06.2018 CN 201810623850
(71) Applicant: The First Affiliated Hospital of Dalian Medical University, Dalian, Liaoning 116011 (CN)
(72) Inventor: LIU, Jing, Dalian, Liaoning 116011 (CN); MA, Jingyun, Dalian, Liaoning 116011 (CN); HAN, Chao, Dalian, Liaoning 116011 (CN)
(74) Representative: Proi World Intellectual Property GmbH
(86) International application number: PCT/CN2019/077105
(87) International publication number: WO 2019/237771

(57) **Abstract**

A stem cell preparation sponge patch complex for treating brain disease, a preparation method therefor and an application thereof, the complex being a degradable sponge soaked in a stem cell suspension, and the stem cells being filled in a suspended state into the pores of a network structure of a sponge material.

## Description

### Technical Field

The present invention belongs to the field of biomedical and tissue engineering research, and specifically relates to a sponge patch complex of stem cell preparation for treating brain diseases, the preparation method and applications thereof.

### Background Art

Brain diseases refer to the brain nerve tissue damage caused by heredity, congenital cerebral hypoplasia, brain trauma, brain tumors, cerebral hemorrhage, brain obstruction, infection, chemical poisoning, etc., covering the brain organic damage of tissue structure and physiological dysfunction. Brain diseases fall into three broad categories, depending on the cause: i) Child cerebral palsy and mental retardation caused by the heredity, congenital dysplasia; ii) The sequelae of acute brain injury caused by trauma and sequela of cerebral apoplexy caused by cerebrovascular disease; iii) chronic degenerative diseases, including Alzheimer's disease, brain atrophy, Parkinson's disease and etc., caused by central nerve injury, senescence and degeneration of brain nerve cells. There are about 10 million new cases of brain diseases in China every year, among which the death and disability rate accounts for about 75%. The national and patients' family medical expenses for brain diseases amount to ten billion Yuan, which has become one of the major diseases endangering the national health.

Brain disease is ultimately nerve tissue damages. Nerve cell, as the basic structure and functional unit of nervous system, due to their hyper-differentiation, once be damaged, some require extremely slow repair process, while others receive irreversible damage. It is a worldwide problem to repair nerve tissue injury and stem cell therapy provides a new approach. Stem cell therapy refers to the separation and purification of autologous, allogeneic or heterogeneous stem cells, which are cultured and amplified in vitro and then transplanted into patients to repair or replace damaged cells or tissues, so as to cure diseases or repair injuries. Studies have shown that certain areas of the nerve center in mammals, including humans, have the potential to regenerate neurons throughout their lives. In the early stage of brain injury, the endogenous neural stem cells (NSCs) in this area can proliferate and migrate to the injured area, and can differentiate into neurons or glial cells in vivo and in vitro, secrete a variety of neurotrophic factors and participate in nerve function repair. How to transplant stem cells into brain efficiently and quickly has become a hot issue in current research. So far, the intracranial transplantation of stem cells has the following ways: i) Lateral ventricular injection, which is not suitable for extensive brain injury because the method mainly implants cells into specific lesions of the lateral ventricle; ii) Transarterial transplantation with low safety; iii) Venous transplantation, in which the rate of cells reaching the treatment area is low; iv) Translumbar puncture transplantation, which is associated with complications.

Intracranial stem cell implantation via the intranasal route has become a new transplantation method which attracts more and more attention. The advantages of transnasal stem cell transplantation include: i) non-invasive; ii) the nasal cavity has a unique direct anatomical connection with the brain, after nasal delivery, drugs absorbed through the nose can bypass the blood-brain barrier into the central nervous system and play a therapeutic role; iii) nasal mucosa is rich in blood vessels and lymphatic vessels, with large absorption area and convenient conditions for entering the intracranial. However, there are some problems with the nasal transplantation: the nasal mucosa has a mucosal ciliary clearance system, which makes the existing dosage forms for nasal drug delivery, including nasal drops and gels, not evenly distributed in the nasal cavity, easy to be lost from the nasal cavity, thereby limiting the absorption of stem cell preparations and the play of their efficacy.

### Summary of the Invention

To solve the above problems, the present invention is disclosed a sponge patch complex of stem cell preparation for treating brain diseases. The complex includes stem cell preparation and sponge material. The composite form is embedded loose combination, so that the stem cells can be loaded and carried by the material, meanwhile, can be released freely under external forces, which is ideal for noninvasive, personalized nasal administration and is expected to be used to improve the current situation of treating brain diseases by nasal administration. A sponge patch complex of stem cell preparation for treating brain diseases, the complex includes a degradable sponge material with an infiltrating stem cell suspension, and the stem cells are filled in the network structure holes of the sponge material in a single cell suspension state.

The degradable sponge material is selected from gelatin, collagen, starch, chitosan, silk fibroin, poly(lactic-co-glycolic acid) polymers, and the compound thereof (such as a compound of collagen and chitosan). In an implementation of the present invention, the sponge material is degradable gelatin sponge.

The sponge patch complex of stem cell preparation can be clamped by the instrument and directly delivered to the optimal physiological site of cell therapy due to its rigid support. For example, the stem cell preparation sponge patch complex can be placed in the olfactory cleft position of the nose using an anterior rhinoscope. This process is non-invasive and can effectively reduce the pain of patients.

In addition to rigidity, the stem cell preparation sponge patch complex is also capable of deformation, which can be fixed at the healing site after delivery. This method can effectively solve the problem of that the distribution of drug in the nasal cavity is not uniform and easy to lost from the nasal cavity due to the mucosal ciliary clearance function of nasal mucosa, specific to existing dosage forms for nasal administration, including nasal drops and gels. This in-situ release of cell preparation can promote the absorption and efficacy of stem cell preparation. In the stem cell preparation sponge patch complex, the cells are loosely bound to the sponge material, and their binding can realize the cell preparation reaching the treatment site under the carrying of sponge materials, and their loose binding can realize the release of cell preparation under the action of external forces, which cannot be achieved by other types of carriers, such as nose drops and gels.

In the stem cell preparation sponge patch complex, the sponge material only plays the role of loading stem cells and carrying them to the treatment site, and then the sponge is biodegraded to effectively avoid foreign body residue.

The stem cell preparation sponge patch complex is biocompatible, avoiding immune rejection and tumorigenicity, having biocompatibility and clinical safety. In practical applications, the shape of gelatin sponge can also be tailored according to the physiological space to be delivered, which makes the complex more suitable for clinical personalized treatment needs and effectively cooperate with adults, children and other patients with different treatment strategies.

For the stem cell preparation sponge patch complex in the present invention, there are many different types of stem cell agents available for the treatment of different brain diseases, such as neural stem cells, embryonic stem cells, mesenchymal stem cells. In an implementation of the invention, the stem cells are preferably neural stem cells. On the one hand, neural stem cells can be cultured in suspension and loaded in the sponge material in the form of single cell suspension. On the other hand, their neuronal differentiation potential and neurotrophic factor secretion can directly promote nerve function repair.

For the neural stem cell suspension culture, the culture medium should select the type that is favorable for single cell suspension culture to prevent cells from attaching to the sponge material which could lead to failure in effective action on the treatment site. In an implementation of the invention, the medium selected is a complete medium for neural stem cells. In the process of suspension culture, with the growth of neural stem cells, they could spontaneously aggregate and grow into spherical clones with uniform morphology. When the spherical clone needs to be processed into scattered cells (single-cell suspension), it can be obtained by collecting, self-precipitation, discarding supernatant, adding digestive juice and mechanically blowing away. Those skilled in the art can also select other ways to obtain single-cell suspension according to experimental conditions.

For the stem cell preparation sponge patch complex in the above technical solution, the density of stem cell suspension is 1×10⁷∼4×10⁵. Under high inoculation density, cells tend to gather in a certain layer of the sponge material, so as to hinder the uniform entry of cells. With the decrease of cell inoculation density, the distribution of cells in the material became more and more uniform. At high inoculation density, dead cells increased. Too little cell density reduces the actual effective concentration of stem cells. After comprehensive consideration and optimization, the density of the stem cell suspension is preferably 1×10⁶∼4×10⁶. Under this inoculation density, the cells can be evenly distributed and live well in the material. In the most optimal case, the cell density of the stem cell suspension is 2×10⁶. In the actual application process, when those skilled in the art determine the cell density, it can also be adjusted according to the actual clinical needs and animal model experiments.

For the stem cell preparation sponge patch complex in the invention, the porosity of biodegradable sponge material is more than 85%. The porosity of the patch material is preferably 97.16%±1.17%. The water absorption rate of the patch material is preferably 98.48%±0.01 %.

Another aspect of the invention is to provide a method for preparing the sponge patch complex of the stem cell preparation. The steps including: take the prepared single cell suspension of neural stem cells, dilute the suspension to the cell density of 1×10⁷∼4×10⁵, and fully infiltrate the degradable sponge material to its absorption saturation using the suspension.

For the preparation method in the present invention, before the full infiltration step of biodegradable sponge material, step of presoaking the degradable sponge material in the medium is also included. After the presoaking step, in order to achieve a more adequate infiltration effect, in general, incubation in close proximity to the cell culture environment is also included, for example, incubation for 1 hour under the condition of 37°C.

Another aspect of the invention is that it discloses the applications of the stem cell preparation sponge patch complex in the preparation of drugs for treating brain diseases. For example, the stem cell preparation sponge patch complex could be used to prepare various sustained release drugs for treating brain diseases.

Beneficial effects of the invention:
1. The complex in the present invention includes stem cell preparation and sponge patch, of which, the cell density of stem cell preparation can be adjusted according to actual demand and the shape of the sponge patch can be tailored according to the physiological space to be served. These make the complex more suitable for clinical personalized treatment needs, and effectively cooperate with different treatment strategies of adults, children and other patients.
2. The stem cell preparation sponge patch complex can be clamped by the instrument and directly delivered to the optimal physiological site of cell therapy due to its rigid support. This process is non-invasive and can effectively reduce the pain of patients.
3. In addition to rigidity, the stem cell preparation sponge patch complex is also capable of deformation, which can be fixed at the healing site after delivery. This method can effectively solve the problem of that the distribution of drug in the nasal cavity is not uniform and easy to lost from the nasal cavity due to the mucosal ciliary clearance function of nasal mucosa, specific to existing dosage forms for nasal administration, including nasal drops and gels. This in-situ release of cell preparation can promote the absorption and efficacy of stem cell preparation.
4. In the stem cell preparation sponge patch complex, the cells are loosely bound to the sponge material, and their binding can realize the cell preparation reaching the treatment site under the carrying of sponge materials, and their loose binding can realize the release of cell preparation under the action of external forces, which cannot be achieved by other types of carriers.
5. The stem cell preparation sponge patch complex in the present invention is characterized and evaluated by material biocompatibility evaluation using the material extraction solution, observation of interaction between the material and cells, investigation of the patch material degradation. The results showed that the extraction solution had no effect on cell proliferation. All the materials were degraded in 21 days of cell culture. It can be seen that the complex system was bio-friendly, avoiding immune rejection and tumorigenicity, and had higher biocompatibility and safety.

### Brief Description of the Drawings

Fig.1 Morphological diagrams of neural stem cells of the same batch and different algebra;
Fig.2 Test result diagrams of the characteristic proteins (Nestin and Sox2) of neural stem cells by flow cytometer;
Fig.3 Growth curve of neural stem cells of different algebra;
Fig.4 Immunofluorescence result diagrams of the characteristic proteins (Nestin and Sox2) of neural stem cells;
Fig.5 Tailor and homogenization of the patch material;
Fig.6 3D display diagram (side view display) of the sponge patch complex sample with neural stem cell seeding density 2×10⁶/mL and culture for 4 days;
Fig.7 3D display diagram (inclination angle display) of the sponge patch complex sample with neural stem cell seeding density 2×10⁶/mL and culture for 4 days;
Fig.8 Growth curve of neural stem cells cultured in material extracts of different concentrations (control, 25%, 50%, 100%);
Fig.9 Observation chart of material and cell interaction on the 4th day of culture;
Fig.10 Degradation curve of sponge materials under cellular action.

### Detailed Discription of the Embodiments

The following is a detailed description of the presnent invention, so as to explain the preparation principle and method of the stem cell preparation sponge patch complex in the present invention without limitation.

### Example 1

Preparation and investigation of stem cell preparation sponge patch complex.

### 1 Preparation and property investigation of neural stem cell preparation

### 1.1 Collection and transportation of tissue sources for neural stem cells

1) The tissue source was the aborted fetus (medical waste) at 8-14 weeks of gestation, and the informed consent was signed for the collection and preservation of donor tissue samples.
2) The container was selected by relevant medical staff according to the size of the tissue, and put into a sterile tube. The mouth of the tube was sealed with sealing film, and the surface of the tube body was wiped and disinfected using alcohol, and then put the tube into the transport box.
3) The transportation process should keep the vehicle stable and avoid the box from being hit. Interior of the transport vehicles should be clean, temperature in the vehicle and transport box should be below 20°C and about 4°C, respectively.

### 1.2 Primary preparation of neural stem cells

1) The intact fetus was placed in a 100 mm sterile petri dish and repeatedly washed with PBS buffer.
2) The skin and bone of the head were stripped, the cranial cavity was opened and brain tissue was exposed, and the vascular membrane around the brain tissue was torn away with fine surgical tweezers.
3) The edge of cerebral cortex was clipped and the cerebral cortex was separated. Then the cerebral cortex was divided into tissue blocks about 1 mm in size in the neural stem cell complete culture medium solution in a 35 mm sterile petri dish.
4) The obtained fetal cerebral cortex and neural stem cell complete medium solution were transferred into a 1 mL centrifuge tube with a dropper, and then slowly blown and sucked for 30 times with a pipette. After 10 minutes of standing, the unblown tissue blocks were precipitated to the bottom of the tube and the supernatant was absorbed.
5) Centrifugation was performed at 820 rpm/min, and the supernatant was absorbed and discarded. 1-2 ml sterile digestive solution was added and blown evenly, and placed in a 35 mm petri dish.
6) Put the dish in an incubator at 37°C and with 5% CO₂ for digestion, after 1 to 2 minutes, add neural stem cells complete medium solution with the same volume for termination of digestion. Centrifugation was performed at 820 rpm/min at 20°C for 5 minutes.
7) The supernatant was removed by a pipette to the top of the cell sedimentation line, and 5 mL of the neural stem cell complete medium solution was added by pipette, and the cells were resuspended by three times of top and bottom blowing. Centrifugation was performed at 820 rpm/min at 20°C for 5 minutes.
8) The supernatant was removed by a pipette to the top of the cell sedimentation line, and 1000 µL of the neural stem cell complete medium solution was added into the centrifuge tube with a 1 mL pipette tip, and gently blown and sucked for 10-15 times to form a single cell suspension and counted.
9) The calculated neural stem cell culture medium was added to the cells, and the cells were blunted into single cell suspensions, seeded at a density of 2-5×10⁵/mL, and added to the cell culture flask. Mark the cell lot number, algebra and cell volume in the upper left of the flask and the operator name and date of operation in the lower right.
10) After inoculation, the cells were observed using an inverted microscope to confirm a uniform distribution in each culture vessel, and if the cells were unevenly distributed, the culture vessel should be shaken again. Record the state of the cells.
11) The cell culture vessel after inoculation and microscopic examination was quickly placed in a carbon dioxide incubator at a culture temperature of 37°C and a CO₂ concentration of 5%.

### 1.3 Neural stem cell passage

1) The neurosphere suspension to be passaged was transferred into a 50 mL centrifuge tube by a pipette, the culture vessel was rinsed with an appropriate amount of PBS buffer, and the rinse solution was transferred to the 50 mL centrifuge tube and allowed to stand for 10 minutes; the cell pellet was collected.
2) Add 1 mL digestive juice to the cells, place the centrifuge tube in a 37°C, 5% CO₂ incubator to digest for 1-2 min; add 4 mL neural stem cell complete medium solution for neutralization; centrifuge at 820 rpm/min, 20°C, for 5 minutes; the supernatant was pipetted into a sterile container and marked, and stored in a -80°C refrigerator as the conditioned medium.
3) The supernatant was pipetted to about 200 µL of the mark, the cell pellet was lightly bombed, and 5 mL of the neural stem cell complete medium solution was added, and centrifuged at 820 rpm/min, 20°C, for 5 minutes.
4) The cells were re-inoculated at a suitable density, and incubated in a 37°C, 5% CO₂ incubator.

### 1.4 Release detection

1) 20-30 mL of cell suspension of stem cell preparation was collected and injected into aerobic blood culture flask, anaerobic blood culture flask and fungal blood culture bottle aseptically, after fully mixing, the opening of the flask/bottle were sealed with a sealing film, and marked cell batch, operator name, operation time, and immediately send for inspection.
2) Three sterile tubes were taken for inspection, 2-3 mL cell culture medium was injected into each tube in a sterile operation mode, the opening of the tube was sealed with sealing film, and marked the cell batch, operator name, operation time, and send them to the clinical lab, for the detection of mycoplasma, chlamydia and pathogenic microorganisms.
3) 2-3 mL of medium aseptically was added to the endotoxin detection tube for endotoxin detection, sealed the opening of the tube with a sealing film, and marked cell batch, operator name, operation time, and immediately send for inspection.
4) All test reports showing negative can be used for warehousing and release operations.

### 1.5 Neural stem cell packaging

1) Cell collection: all samples in the culture flask were aspirated into a 50 mL centrifuge tube, centrifuged at 820 rpm/min for 5 minutes at 20 °C.
2) Preparation of single cell suspension: the supernatant was pipetted to about 200 µL of the mark, 2 mL of neural stem cell complete medium solution was added, and repeatedly blowed and sucked the cell pellet for 30 times with a 1 mL pipette tip until physically to single cell suspension, which then was centrifuged at 820 rpm/min for 5 minutes at 20 °C.
3) Cell count: the supernatant was aspirated, 1 mL of PBS buffer was added, the bottom of the tube was flicked to disperse and mix the cells, and was gently blowed 3-5 times with a pipette, and 10 µL of single cell suspension was taken for counting.
4) Cell density adjustment: according to the counting result, the concentration of the cell suspension was adjusted to the target concentration by adding an appropriate amount of PBS buffer.
5) Cell sieving: another sterile 50 mL centrifuge tube was prepared, the cell sieve was clamped with a tweezers and was placed on the centrifuge tube. The cell suspension with adjusted density was absorbed and collected through the cell sieve into the prepared centrifuge tube.
6) Cell packing and sampling: the cell suspension was added to the prepared 2 mL cryotube, the cap was screwed and sealed with the sealing film. At the same time, 0.5 mL cell suspension was added into a 2 mL cryotube, and was marked and sealed with a sealing film, and frozen it at -80°C for as retention samples for inspection.
7) Cell preparation labeling: the prepared cell preparations were labeled and placed in a refrigerator at 4°C for temporary storage.

### 1.6 Neural stem cell identification

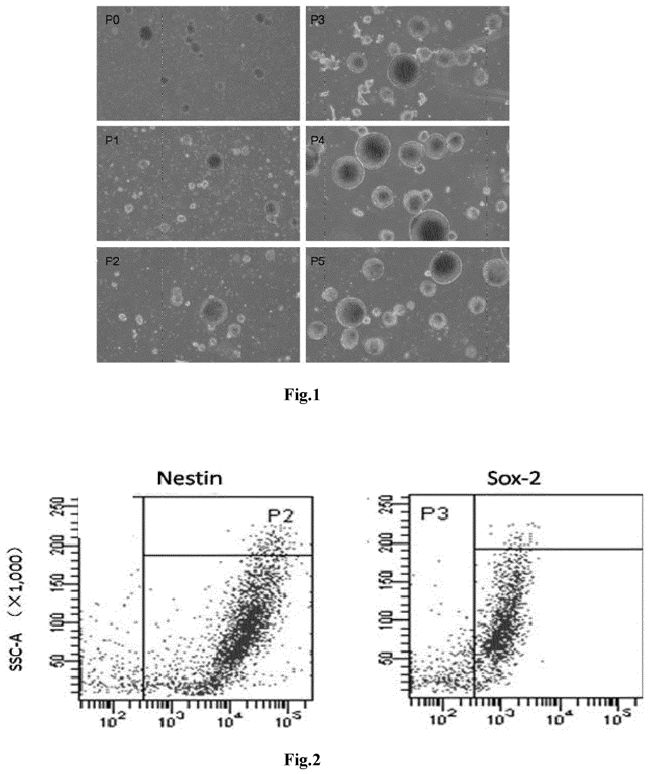

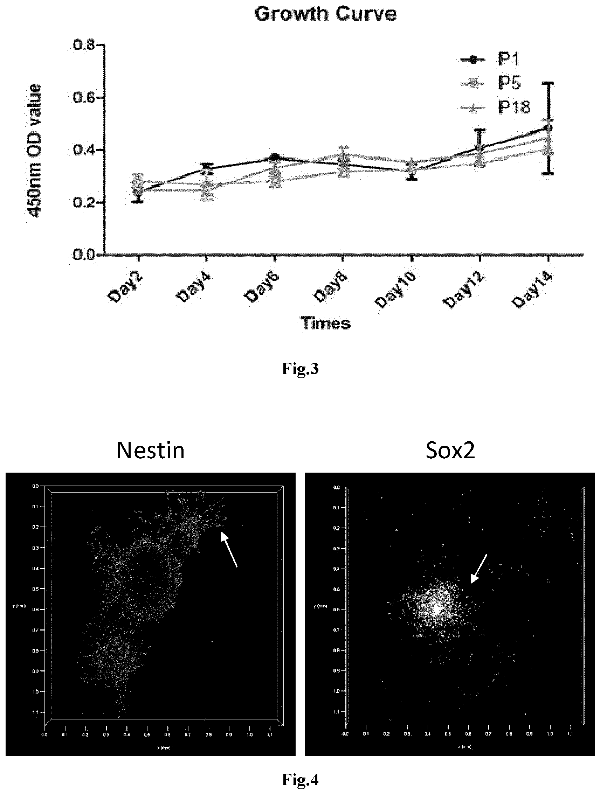

1) Cell morphology detection: The individual cells of the neural stem cells were spherical; under single cell inoculation, as the cells grew, they spontaneously aggregated and grew into a spherical clone with uniform morphology. Morphological diagrams of neural stem cells of the same batch and different algebra are shown in Fig.1.
2) Cell marker expression by flow cytometer detection: 1×10⁶ cells were taken for detection, and the cells expressed characteristic proteins Nestin and Sox2 of neural stem cell. Test results by flow cytometer are shown in Fig.2. The Nestin+/Sox2+ cell ratio is greater than 90%.
3) Cell proliferation capacity: 100 µL of neural stem cell suspensions of different culture batches and different algebra were added to a 96-well plate at a cell concentration of 1000 cells/well. The plate was incubated for 2-14 days in the incubator, 10 µL of CCK-8 solution was added to each well, the plate was incubated for 2 h in the incubator, and measured the absorbance at 450 nm using a microplate reader. Using the numerical values, a curve with the culture time as the abscissa (X-axis) and the OD value as the ordinate (Y-axis) was created. Based on this curve, the doubling time of the cells can be calculated to reflect the proliferative capacity of the cells (Fig.3).
4) Cell marker immunofluorescence assay: Human neural stem cell suspension was taken and washed for 3 times with PBS, and fixed with paraformaldehyde. The neural stem cell-specific markers Nestin and Sox2 were stained with as shown in Fig.4.

### 1.7 Cell seeding

1) A corresponding volume of neural stem cell culture medium is added depending on the type of cell culture vessel used. 2 mL per well was added to the low-adsorption 6-well plate, 5 mL/vial was added to the T25 cell culture flask, 15 mL/vial was added to the T75 cell culture flask, and 50 ml/vial was added to the T175 cell culture flask.
2) The counted cell suspension was added to the culture vessel, and the container of the original cell suspension was washed with an appropriate amount of the medium, and transferred in. A certain volume of cell culture medium was added and the cell suspension was shaken manually. A suitable range of pipette was used to blow under the liquid surface, the cell lot number and cell mass were marked on the upper left side of the culture flask, and the operator name and operation date were marked on the lower right side.
3) It was confirmed by observation under an inverted microscope that the cells were roughly distributed uniformly in each culture container, and if the cells were unevenly distributed, the container was shaken again. Images of cell status and media status were recorded every two days. This microscopic examination should be completed in 5 minutes.
4) The cell culture vessel after inoculation and microscopic examination was quickly placed in a 37°C, 5% CO₂ incubator. Pay attention to keeping the level of the container while moving. If it is a well plate, keep the plate covered during the movement. If the container is a culture bottle, the bottle opening should be left or right instead of outward when placed in a carbon dioxide incubator. The container should not be placed close to the door, but should be slightly inward to reduce the risk of contamination when opening and closing the door.

### 2 Preparation and performance investigation of the sponge patch material

### 2.1 Cutting and homogenization of patch materials

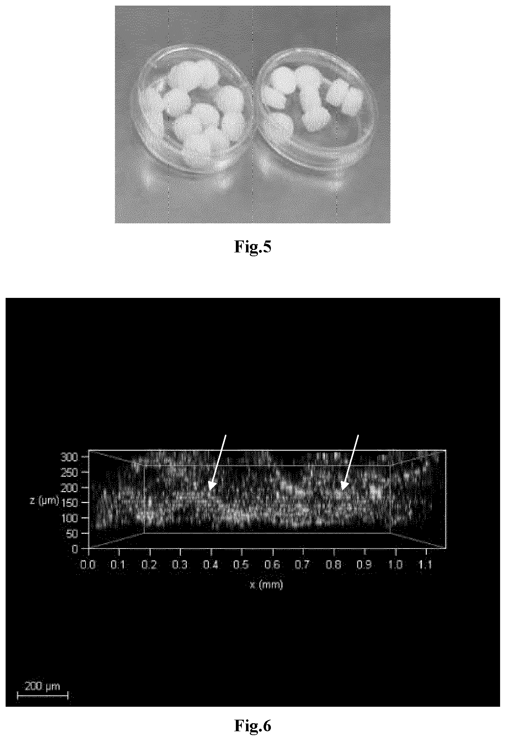

In the ultra-clean platform, the medical degradable gelatin sponge was taken, and the material was cut with an 8 mm circular puncher; the cut material was a cylinder with a diameter of 8 mm and a thickness of 5 mm (Fig.5). No weighing is required to ensure that the mass difference between each sponge sample is within 0.0004g, and also, no secondary sterilization is required.

### 2.2 Determination of patch material porosity

Anhydrous ethanol was taken into the measuring cylinder, the volume was recorded as V1; the patch material was immersed, completely saturated, the volume was recorded as V2; the patch material was taken out, and the remaining volume was recorded as V3; the porosity of the patch material was calculated as δ=(V1-V3)/(V2-V3) x100%; three samples were taken from the above measurement, and the average value and standard deviation were calculated: the porosity of the patch material was 97.16% ± 1.17%.

### 2.3 Determination of water absorption rate of patch materials

Weighing method was used to determine the water absorption rate of the patch material. The dry weight of the material was recorded as W1; the material was immersed into the ultra-pure water for 24 h and completely saturated, the material was taken out and removed the surface moisture, and this weight of the material was recorded as W2; the calculation formula: θ=[(W2-W1)/W2] ×100%; three samples were taken from the above measurement, and the average value and standard deviation were calculated: the water absorption rate of the patch material was 98.48%±0.01%.

### 3 Preparation and performance investigation of the stem cell preparation sponge patch complex

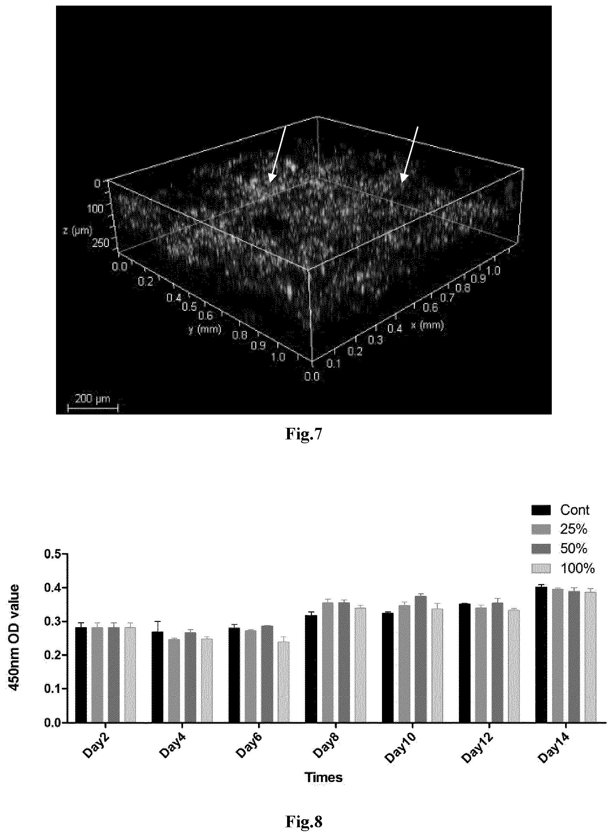

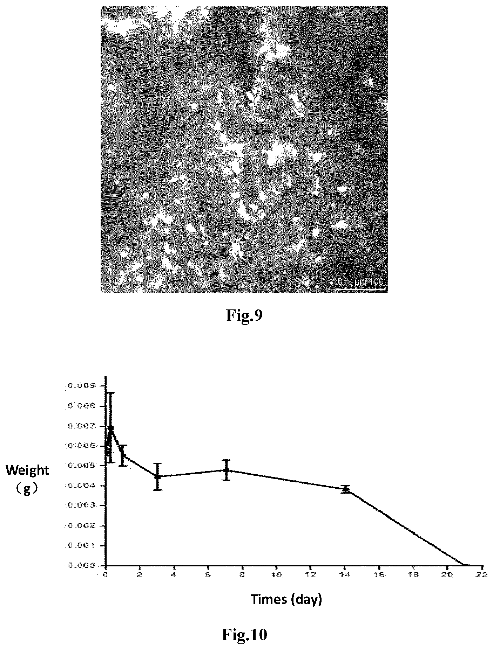

3.1 Determination of the mode of cell loading with material: a cylindrical gelatin sponge been cut with a diameter of 8 mm and a thickness of 5 mm was placed into a 24-well culture plate, and pre-soaked with medium; a pre-prepared single-cell suspension of neural stem cells was inoculated with 250 µL per well; after 1 hour of incubation, the material was applied. The following seeding of cells on the material was all in this manner.
3.2 Determination of cell-loading capacity of the material: cells were seeded in the material, and the cell density of 1-4 groups was set at 5×10⁷, 1×10⁷, 2×10⁶, 4×10⁵/mL, respectively; the sponge was taken out, cells that were not attached to the sponge during cell seeding were collected by saline. The remaining liquid volume and cell concentration were counted, and the remaining cell number was calculated. According to the adsorption rate=the number of adsorbed cells/the number of original cells, the cell adsorption rates of 1-4 groups were calculated as: 97.4%, 98.5%, 96.5%, and 93.2%, respectively.
3.3 The cell seeding density was optimized by the distribution and activity of cells in the material: in order to achieve uniform distribution and good survival of the cells in the material, the distribution and activity of the cells at different seeding densities were investigated. Sponge patch composite samples with neural stem cell cultured for 4 days was selected, and the cell seeding density of 1-4 groups was set at 5×10⁷, 1×10⁷, 2×10⁶, 4×10⁵/mL, respectively; the original medium was discarded, dipped twice with PBS buffer, Live/Dead dye solution (Calcein-AM/PI) was added to immerse the material, placed in 37°C, 5% CO₂ incubator for half an hour, observed and took photos under confocal microscope; cell distribution and survival were recorded, among them, fluorescence represented the live and death staining of cells (Fig.6 and 7). It was found that under high seeding density, cells tend to accumulate at a certain level of the material, which hinders the uniform entry of cells; as the cell seeding density decreases, the distribution of cells in the material becomes more and more uniform; at high seeding density, the number of dead cells increases; when the cell density is too low, the actual effective concentration of stem cells could be reduced. Taken together, the preferred cell seeding density was determined to be 2×10⁶/mL.
3.4 Detection of biocompatibility of materials using material extracts: A cylindrical gelatin sponge with a diameter of 8 mm and a thickness of 5 mm was placed into a culture flask, added with neural stem cell culture medium, and placed in a 37°C incubator for 24 h to prepare a gelatin sponge extract; medium solutions containing the material extract concentration of 100%, 50%, 25% and 0% were prepared respectively, chilling at 4 ° C to set aside; the cells were seeded into a 96-well plate at a density of 2×10⁶/mL, and the above four concentrations of the material extracts were separately added. The cells were placed in a 37°C, 5% CO₂ incubator for cultivation. The experimental groups were set as 2 d, 4 d, 6 d, 8 d, 10 d, 12 d, and 14 d group. At the set time point, the original medium was discarded and 100 µL of the corresponding medium and 10 µL of CCK-8 solution were added. The cells were incubated in the incubator for 2 hours, and the absorbance was measured by a microplate reader. Growth curve (Figure 8) obtained by CCK-8 measurement shows that different groups of extracts had no effect on cell proliferation (no statistical difference).
3.5 Observation of interaction between material and cells: Selected sponge patch composite samples with neural stem cell cultured for 4 days were seeded with density of 2×10⁶/mL; confocal photographing analysis was performed; evaluation was performed by overlaying images of cells and materials, in which fluorescence represented the live/death staining of cells, and the bright field showed the network structure of the material. The results are shown in Fig.9. The binding of the cells to the material is a chimeric loose binding that does not adhere to the network structure of the sponge; that is, the cell suspension is filled in the pores of the material network structure.
3.6 Investigation on in vitro degradation of patch materials: 21 samples of sponge were taken and inoculated neural stem cells at 2×10⁶/mL for continuous culture; at each time point of 0 h, 6 h, 1 d, 3 d, 7 d, 14 d, and 21 d, 3 samples were taken; after washing three times with ultrapure water, the samples were dried at 40°C or used filter paper for treatment, and weighed them. Determine the degree of biomaterial degradation by calculating the remaining weight, as shown in Fig.10. Under the effect of cells, the quality of gelatin sponge material was greatly improved compared with the original quality at 6 h, due to the swelling of the gelatin material; subsequently, the quality decreased; at 21 d, the samples were gelatinous, all dissolved after washing, to achieve complete degradation.

## Claims

1. A sponge patch complex of stem cell preparation for treating brain diseases, wherein the complex comprises a degradable sponge material with an infiltrating stem cell suspension, and the stem cells are filled in the network structure holes of the sponge material in a single cell suspension state.

2. The sponge patch complex of stem cell preparation according to claim 1, wherein the degradable sponge material is selected from gelatin, collagen, starch, chitosan, silk fibroin, poly(lactic-co-glycolic acid), and the complex thereof.

3. The sponge patch complex of stem cell preparation according to claim 1, wherein the stem cells are neural stem cells.

4. The sponge patch complex of stem cell preparation according to claim 1, wherein the cell density of the stem cell suspension is 1×10⁷∼4×10⁵.

5. The sponge patch complex of stem cell preparation according to claim 4, wherein the cell density of the stem cell suspension is 1×10⁶∼4×10⁶.

6. The sponge patch complex of stem cell preparation according to claim 1, wherein the porosity of the degradable sponge material is more than 85%.

7. A method for preparing the sponge patch complex of stem cell preparation claimed in claim 1, comprising: diluting a prefabricated single cell suspension of neural stem cells to a cell density of 1×10⁷∼4×10⁵, fully infiltrating the degradable sponge material into the single cell suspension until saturating.

8. The method for preparing the sponge patch complex of stem cell preparation according to claim 7, wherein before the full infiltration of the degradable sponge material, step of pre-soaking the degradable sponge material in the medium is also comprised.

9. The method for preparing the sponge patch complex of stem cell preparation according to claim 8, wherein the medium is a complete medium for neural stem cells.

10. An application of the stem cell preparation sponge patch complex according to claim 1 in the preparation of drugs for the treatment of brain diseases.
